# EUROPÄISCHE PATENTSCHRIFT

(11) **EP 1 369 144 B1**
(45) Veröffentlichungstag und Bekanntmachung des Hinweises auf die Patenterteilung: **29.06.2005**
(21) Anmeldenummer: 03011293.2
(22) Anmeldetag: 17.05.2003
(51) Int. Cl.: A61N 1/39

(54) **Defibrillator**
Defibrillator
Défibrillateur

(30) Priorität: 06.07.2002 DE 10230594; 12.12.2002 DE 10258010
(43) Veröffentlichungstag der Anmeldung: 10.12.2003
(73) Patentinhaber: Weinmann Geräte für Medizin GmbH & Co. KG, 22525 Hamburg (DE)
(72) Erfinder: Griefahn, Joachim, Dr., 22523 Hamburg (DE)
(74) Vertreter: Klickow, Hans-Henning, Dr.-Ing.

(56) Entgegenhaltungen:
- DE-A- 3 614 872
- DE-A- 19 841 070
- DE-C- 19 931 012
- US-A- 5 632 268
- US-A- 5 848 591
- US-B1- 6 327 497
- US-B1- 6 351 671

## Beschreibung

Die Erfindung betrifft eine Vorrichtung zur Defibrillation, die einen Spannungsgenerator, mindestens zwei mit dem Spannungsgenerator verbundene Elektroden, eine Steuerung zur Funktionskoordinierung sowie eine Beatmungseinrichtung mit Abgabeelement aufweist und bei der die Beatmungseinrichtung mit einer Druckgasflasche für Sauerstoff und einem zwischen der Druckgasflasche und dem Abgabeelement angeordneten Dosierelement versehen ist sowie bei der das Dosierelement an eine Steuerung zur Durchführung einer Oxigenierung angeschlossen ist.

Entsprechende Vorrichtungen werden insbesondere im Zusammenhang mit einer Notfallbehandlung von Patienten eingesetzt, bei denen ein akutes Herzversagen vorliegt. Über den Defibrillator wird ein Elektroschock erzeugt, der das Herz des Patienten dazu veranlassen soll, aus dem Fibrillations-Zustand herauszugelangen. Die Beatmung des Patienten unter Verwendung des Abgabeelementes beispielsweise einer Atemmaske oder einer Sauerstoffbrille dient dazu, Gehirnschäden zu verhindern oder wenigstens zu vermindern, die aufgrund einer ungenügenden Sauerstoffversorgung des Gehirns in Folge des Fibrillations-Zustandes des Herzens hervorgerufen wird.

Ein besonderes Problem liegt darin, daß sowohl bei einer Diagnose des Fibrillations-Zustandes als auch während der Defibrillation keine Beatmung durchgeführt werden darf. Eine Beatmungsdurchführung während der Diagnose beeinträchtigt eine eindeutige Erkennung des Fibrillations-Zustandes, eine Durchführung der Beatmung während des Defibrillationsvorganges verursacht ein Risiko von Artefakten. Diese Probleme führen dazu, daß in der Regel eine Beatmung erst nach der Diagnose des Fibrillations-Zustandes und der Durchführung der Defibrillation begonnen wird.

Als Folge dieses üblichen Vorgehens erleiden eine erhebliche Anzahl von Patienten Gehirnschädigungen, da bereits nach 3 Minuten einer fehlenden oder unzureichenden Sauerstoffzufuhr Gehirnzellen geschädigt werden bzw. absterben.

Ein zusätzliches Problem besteht darin, daß Fibrillations-Zustände in der Regel nicht bei Anwesenheit von fachkundigem medizinischen Personal, sondern während des normalen Alltagslebens eintreten. Bereits existierende mobile Defibrillatoren erweisen sich insbesondere im Zusammenhang mit der auftretenden Atmungsproblematik aber als nur sehr unzureichend dafür geeignet, von medizinisch nicht ausgebildeten Personen bedient zu werden.

In der US-B-6,327,497 werden ein Verfahren und eine Vorrichtung zur Steuerung eines Defibrillators beschrieben, bei denen über einen Spannungsgenerator eine Elektroschockspannung an Elektroden angelegt wird. Zusätzlich wird von einer Druckgasflasche Sauerstoff zu einem Abgabeelement geleitet. Die Sauerstoffabgabe wird von einem Dosierelement zur Durchführung einer Oxygenierung gesteuert.

Aus der US-A-5,632,268 ist ein transportables System zur Sauerstoffversorgung eines Patienten bekannt. Der Sauerstoff wird einer Druckgasflasche entnommen.

In der DE-A-36 14 872 wird eine Einrichtung zur Mischung von Luft und Sauerstoff beschrieben, wobei die Mischung einer Nasalmaske zugeführt wird.

Aus der DE-A-198 41 070 ist eine Sauerstoffbrille bekannt, um einem Patienten nasal Sauerstoff zuzuführen.

Die DE-A-199 31 012 beschreibt einen manuell betätigbaren Beatmungsbeutel zur Atemgasversorgung eines Patienten.

In der US-A-5,848,591 wird eine Beatmungseinrichtung beschrieben, bei der von einem Gebläse erzeugte Atemluft über eine Druckgasflasche bereitgestellter Sauerstoff zugemischt werden kann, um einen erhöhten Sauerstoffgehalt der Atemluft zu erreichen.

Aufgabe der vorliegenden Erfindung ist es, eine Vorrichtung der einleitend genannten Art derart zu konstruieren, daß eine kurzfristige Versorgung des Patienten mit Sauerstoff unterstützt wird.

Diese Aufgabe wird erfindungsgemäß dadurch gelöst, daß die Steuerung eine Zeitsynchronisation zur zeitlich aufeinander folgenden Durchführung von Inspirationsphasen und Exspirationsphasen, einer Fibrillationsanalyse sowie einer anschließenden Defibrillation bei Erkennung eines Fibrillations-Zustandes aufweist.

Die Kombination des Defibrillators mit einer Einrichtung zur Oxigenierung sowie das entsprechende Verfahren zur Gerätesteuerung ermöglichen es, daß unmittelbar bei Erkennung eines Fibrillations-Zustandes oder auch bereits lediglich bei der Erkennung eines eventuellen Fibrillations-Zustandes bzw. einer Vorstufe hierzu eine Oxigenierung des Patienten eingeleitet wird.

Die Oxigenierung mit einem geringen Volumenfluß an reinem Sauerstoff kann sowohl während einer eventuellen ärztlichen Diagnose des Zustandes des Patienten als auch bei einer möglichen Applikation von Elektroschocks durchgeführt wer den. Es wird weder die Genauigkeit der Diagnose vermindert noch liegt bei der Applikation von Elektroschocks ein erhöhtes Risiko von Artefakten vor.

Das Verfahren zur Steuerung des Defibrillators ermöglicht es insbesondere auch für medizinisch ungebildete Ersthelfer, das Gerät zur Sauerstoffversorgung des Patienten zu aktivieren, ohne daß das Risiko einer zusätzlichen Schädigung des Patienten vorliegt. Der Ersthelfer braucht hierzu dem Patienten lediglich das Abgabeelement, beispielsweise eine Beatmungsmaske, anlegen und die Oxigenierungsfunktion des Gerätes aktivieren.

Zur Gewährleistung einer ausreichend langen Betriebsfähigkeit wird vorgeschlagen, daß für die Oxigenierung die Do-sierungsmenge etwa 1000ml bis 1500ml reiner Sauerstoff je Minute beträgt.

Eine typische Realisierung besteht darin, daß als Abgabeelement eine Nasalmaske verwendet wird.

Eine optimale Koordinierung zwischen der Sauerstoffabgabe und der Defibrillation erfolgt dadurch, daß durch die Strömung von reinem Sauerstoff eine Oxigenierung durchgeführt wird.

Eine weitere Optimierung der Funktionskoordinierung wird dadurch erreicht, daß eine meßtechnische Überwachung von Inspirationsphasen und Exspirationsphasen bei einer Atemgasversorgung durchgeführt wird.

Die Verwendung einer einfachen Gerätetechnik wird dadurch unterstützt, daß zur Erzeugung eines Beatmungsdruckes bei der Sauerstoffversorgung ein Beatmungsbeutel verwendet wird.

Eine Anwendung durch ungeübte Benutzer wird dadurch unterstützt, daß aufeinander folgend mehrere Inspirationsphasen mit reinem Sauerstoff sowie mehrere Exspirationsphasen durchgeführt werden.

Insbesondere können Bedienungsfehler dadurch vermieden werden, daß die Steuerung nach einer Aktivierung vollautomatisch aufeinander folgende Inspirationsphasen mit reinem Sauerstoff sowie Exspirationsphasen vorgibt.

Eine kurzfristige Oxigenierung wird dadurch unterstützt, daß die Inspirationsphasen und die Exspirationsphasen mit einer Dauer von jeweils etwa zwei Sekunden vorgegebenen werden.

Für eine wirksame Oxigenierung erweist es sich als ausreichend, daß etwa vier bis fünf aufeinander folgende Inspirationsphasen und Exspirationsphasen vorgegeben werden.

Ein vorteilhaftes Steuerungskonzept wird dadurch bereitgestellt, daß die Steuerung nach der aufeinander folgenden Durchführung von Inspirationsphasen und Exspirationsphasen einen Analysator zur Detektion eines möglichen Fibrillationszustandes aktiviert.

Zur Realisierung der vorgesehenen Strömungsmenge wird vorgeschlagen, daß das Dosierelement als ein Durchflußbegrenzer ausgebildet ist.

Zur Unterstützung eines automatischen Betriebes erweist es sich als zweckmäßig, daß der Defibrillator einen Detektor aufweist, der an die Elektroden angeschlossen ist.

Eine weiter vereinfachte Bedienungsstruktur wird dadurch realisiert, daß sowohl der Defibrillator als auch eine Steuerung für die Druckgasflasche an einen übergeordneten Koordinator angeschlossen sind.

Eine vorteilhafte vollautomatische Gerätesteuerung wird insbesondere dadurch realisiert, daß die Steuerung eine Zeitsynchronisation zur zeitlich aufeinander folgenden Durchführung von Inspirationsphasen und Exspirationsphasen, einer Fibrillationsanalyse sowie einer anschließenden Defibrillation bei Erkennung eines Fibrillationszustandes aufweist.

In den Zeichnungen sind Ausführungsbeispiele der Erfindung schematisch dargestellt. Es zeigen:
- Fig. 1: eine perspektivische Darstellung eines Defibrillators mit separater Sauerstoffquelle,
- Fig. 2: ein schematisches Blockschaltbild zur Veranschaulichung der Gerätefunktion,
- Fig. 3: eine schematische Darstellung einer Beatmungseinrichtung mit Beatmungsbeutel und
- Fig. 4: eine schematische Darstellung einer Beatmungseinrichtung, bei der konzentrierter Sauerstoff von einer Druckgasflasche gesteuert über einen Beatmungsbeutel einer Atemmaske zuführbar ist.

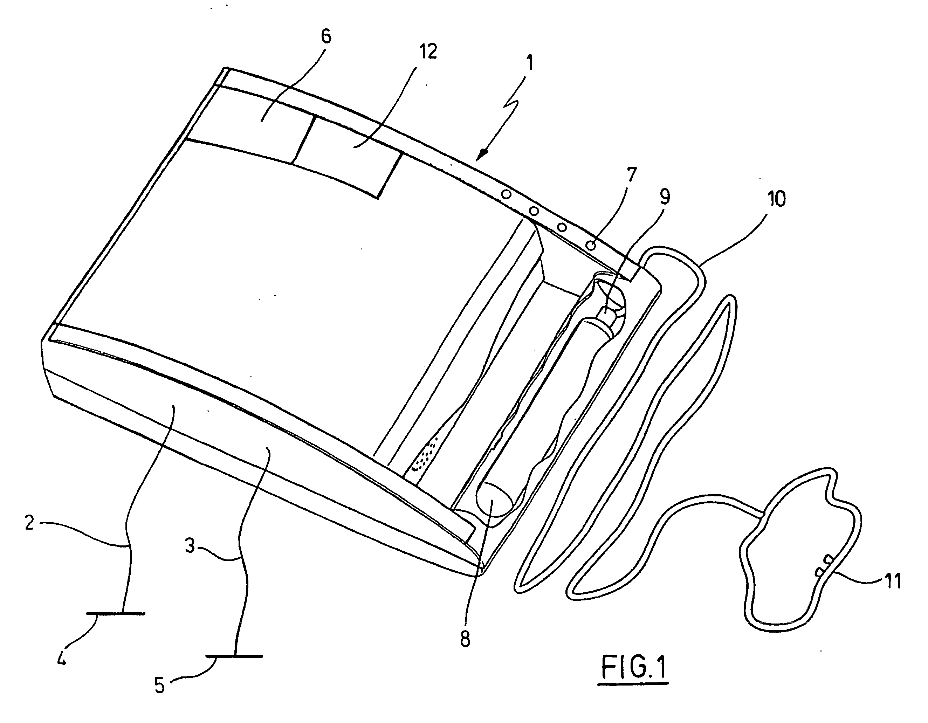

Fig. 1 zeigt in einer Prinzipdarstellung eine Ansicht eines Defibrillators (1), der über Verbindungsleitungen (2, 3) mit Elektroden (4, 5) verbunden ist. Der Defibrillator (1) ist mit einer Steuerung (6) versehen und über einen Schalter (7) aktivierbar.

Fig. 1 zeigt darüber hinaus eine Druckgasflasche (8) mit reinem Sauerstoff, die über ein Dosierelement (9) sowie einen Beatmungsschlauch (10) mit einem Abgabeelement (11) verbunden ist. Das Dosierelement (9) ist von einer Steuerung (12) aktivierbar. Das Abgabeelement (11) ist als eine Sauerstoffbrille ausgebildet.

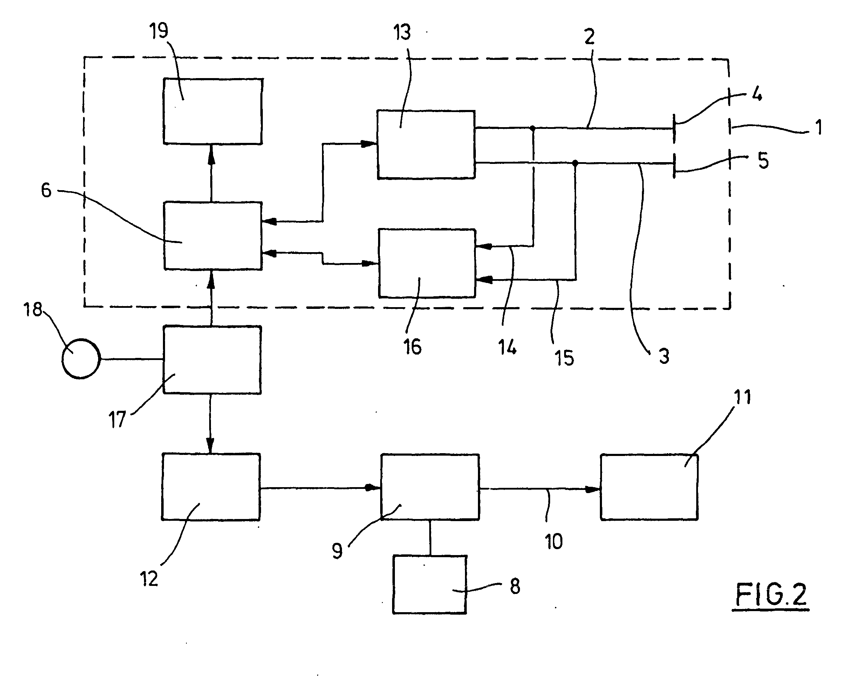

Das Blockschaltbild in Fig. 2 zeigt den Defibrillator (1) mit der Druckgasflasche (8) als Atemgasquelle in einer Darstellung als schematisches Blockschaltbild. Insbesondere ist zu erkennen, daß die Verbindungsleitungen (2, 3) an einen Spannungsgenerator (13) als Schockgeber angeschlossen sind. Der Spannungsgenerator (13) seinerseits ist mit der Steuerung (6) verbunden. Zur Ermöglichung einer Erkennung eines Fibrillations-Zustandes sind die Elektroden (4, 5) über Meßleitungen (14, 15) mit einem Detektor (16) verbunden, der zur-Aufnahme eines Elektrokardiogramms ausgebildet ist. Der Detektor (16) übermittelt die Meßwerte bzw. aus den Meßwerten abgeleitete Diagnoseinformationen an die Steuerung (6).

Insbesondere ist auch daran gedacht, den Detektor (16) dafür einzusetzen, einen optimalen Zeitpunkt für die Generierung der Elektroschocks durch den Spannungsgenerator (13) vorzugeben. Eine entsprechende Betriebsweise wird durch den Anschluß sowohl des Detektors (16) als auch des Spannungsgenerators (13) an die Steuerung (6) unterstützt.

Bei der in Fig. 2 dargestellten Ausführungsform sind sowohl die Steuerung (12) der Beatmungseinrichtung als auch die Steuerung (6) des Defibrillators (1) an einen Koordinator (17) angeschlossen, der mit einem gemeinsamen Hauptschalter (18) gekoppelt ist. Eine derartige Konstruktion erleichtert insbesondere eine Anwendung des Gerätes durch medizinische Laien.

Eine Anwendung des in Fig. 2 skizzierten Gerätes kann beispielsweise derart erfolgen, daß bei einer Ausbildung als mobiles Notfallgerät nach Erkennung eines möglichen Fibrillations-Zustandes durch einen anwesenden Ersthelfer in einem ersten Schritt das Abgabeelement (11) beim Patienten angelegt und über den Hauptschalter (18) die Oxigenierung eingeschaltet wird, um Hirnschädigungen beim Patienten durch mangelhafte Sauerstoffversorgung vorzubeugen. Die Sauerstoffversorgung erfolgt in Form einer Oxigenierung mit reinem Sauerstoff und einer Strömungsmenge je Zeiteinheit im Bereich vom 1000 ml/min bis 1500 ml/min an reinem Sauerstoff.

In einem nächsten Schritt werden dem Patienten dann die Elektroden (4, 5) angelegt und der Detektor (16) kann eine konkrete Analyse vornehmen. Stellt sich dabei heraus, daß der vermutete Fibrillations-Zustand nicht vorliegt, so kann über eine Anzeigeeinheit (19) visuell und / oder akustisch eine zugehörige Meldung generiert werden. Für den Fall einer Erkennung eines konkreten Fibrillations-Zustandes wird von der Steuerung (6) über den Spannungsgenerator (13) und die Elektroden (4, 5) ein Elektroschock appliziert. Die entsprechende Wirkung kann wieder über den Detektor (16) überprüft werden, so daß der Ablauf gegebenenfalls wiederholt wird. Auch während der Durchführung der Defibrillation erfolgt kontinuierlich weiter die Oxigenierung unter Verwendung des reinen Sauerstoffes.

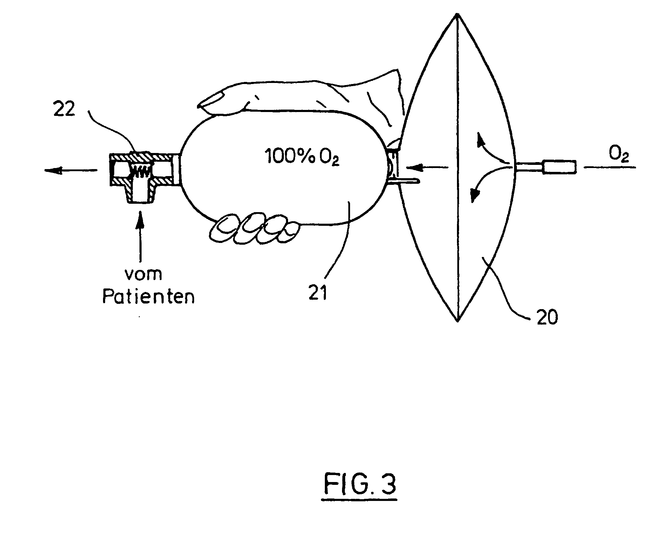

Fig. 3 zeigt eine Ausführungsvariante, bei der von einer nicht dargestellten Druckgasflasche (8) einem Reservoirbeutel (20) reiner Sauerstoff zugeführt wird. Der Reservoirbeutel (20) ist an einen Beatmungsbeutel (21) angeschlossen, der bei einer manuellen Druckbeaufschlagung den Sauerstoff in Richtung auf den Patienten pumpt. Bei einer Beendigung der manuellen Kontraktion des Beatmungsbeutels (21) strömt die Luft vom Patienten über ein Ausatemventil (22) in eine Umgebung. Mit Hilfe des Beatmungsbeutels (21) ist es möglich, auch bei einem vollständigen oder zumindest teilweisen Versagen einer Eigenatmung des Patienten Sauerstoff in die Lunge des Patienten zu pumpen und hierdurch für eine ausreichende Oxigenierung zu sorgen. Typischerweise erfolgen zunächst vier bis fünf manuelle Hübe zur Gewährleistung einer ausreichenden Sauerstoffversorgung des Patienten, bevor eine Analyse eines möglichen Fibrillation-Zustandes sowie gegebenenfalls eine anschließende Defibrillation eingeleitet wird.

Alternativ zur Verwendung des Beatmungsbeutels (21) ist es auch möglich, ein Demand-System mit Reservoirbeutel (20) zu verwenden oder eine Steuerung unter Verwendung einer Auslösetaste zu realisieren.

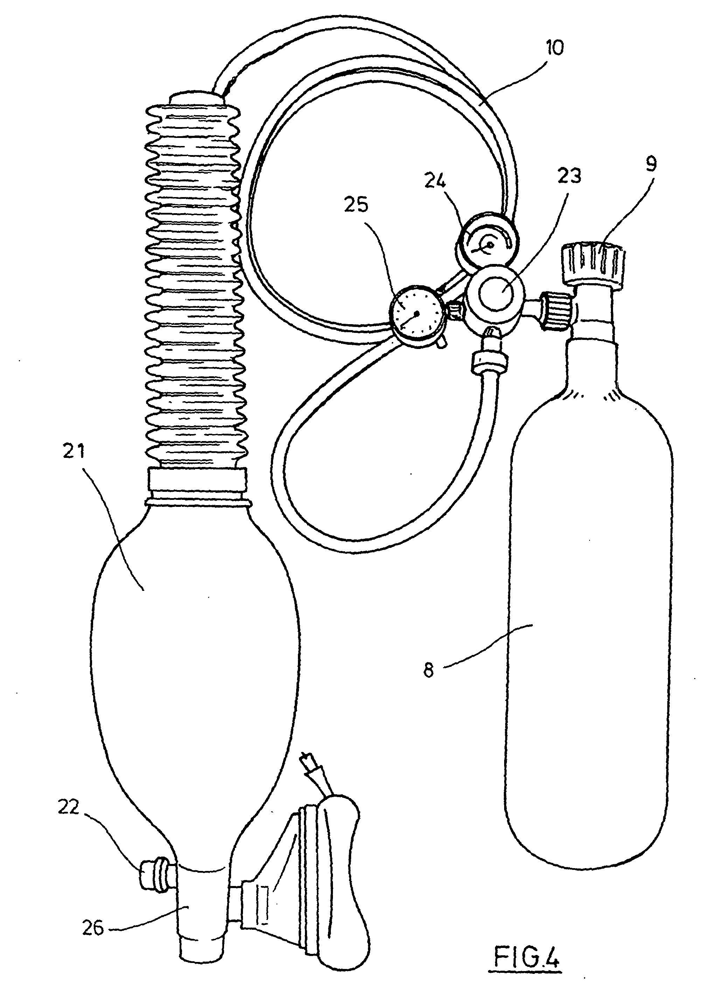

Fig. 4 zeigt eine Ausführungsvariante, bei der die Druckgasflasche (8) über das Dosierelement (9) und eine Betätigungseinrichtung (23) an den Beatmungsschlauch (10) sowie das als Atemmaske ausgebildete Abgabeelement (11) angeschlossen ist. Über eine entsprechende manuelle Auslösung der Betätigungseinrichtung (23) kann hier die Oxigenierung des Patienten durchgeführt werden.

Das Dosierelement (9) ist typischerweise als ein Drehventil ausgebildet. Typischerweise wird die Betätigungseinrichtung (23) seitlich an das Dosierelement (9) angeschraubt. Zur Drucküberwachung sowohl vor als auch hinter einer Druckreduzierung werden zwei Manometer (24, 25) eingesetzt. Auch bei der in Figur 4 gezeigten Ausführungsform ist zwischen dem Beatmungsschlauch (10) und dem als Beatmungsmaske ausgebildeten Abgabeelement (10) ein Beatmungsbeutel (21) angeordnet. Typischerweise ist das Abgabeelement (12) im Bereich eines Anschlußstutzens (26) des Beatmungsbeutels (21) angeordnet, der einer Einmündung des Beatmungsschlauches (10) in den Beatmungsbeutel (21) abgewandt angeordnet ist. Im Bereich des Anschlußstutzens (26) ist ebenfalls das Ausatemventil (22) positioniert.

Gemäß einer vollautomatischen Ausführungsvariante ist daran gedacht, daß von einem Notfallhelfer dem Patienten lediglich als Abgabeelement (11) die Atemmaske und die Elektroden (4, 5) angelegt werden und daß der gesamte weitere Ablauf vollautomatisch nach einer Aktivierung der Anlage durchgeführt wird. Hierdurch können Fehlbedienungen durch ungeübte Ersthelfer weitgehend vermieden werden.

Ein entsprechender vollautomatischer Ablauf wird derart durchgeführt, daß zunächst vier bis fünf Oxigenierungszyklen durchlaufen werden, bei denen dem Patienten unter Druck reiner Sauerstoff in die Lunge hineingedrückt wird. In der Regel ist es ausreichend, vier bis fünf Beatmungshübe mit jeweils zwei Sekunden Inspirationsdauer und zwei Sekunden Exspirationsdauer durchzuführen, um eine sehr hohe Sauerstoffsättigung in der Lunge bereitzustellen. Nach somit etwa sechzehn bis zwanzig Sekunden ist für eine ausreichende Oxigenierung des Patienten gesorgt, so daß eine weitere Beatmung zunächst beendet und eine Analyse eines möglichen Fibrillationszustandes erfolgen kann. Eine entsprechende vollautomatische Gerätesteuerung schließt somit Fehlbedienungen weitgehend aus und sichert eine kurzfristige Oxigenierung des Patienten, um Hirnschäden durch mangelnde Sauerstoffversorgung zu vermeiden.

## Patentansprüche

1. Vorrichtung zur Defibrillation, die einen Spannungsgenerator (13), mindestens zwei mit dem Spannungsgenerator (13) verbundene Elektroden (4, 5), eine Steuerung zur Funktionskoordinierung sowie eine Beatmungseinrichtung mit Abgabeelement aufweist und bei der die Beatmungseinrichtung mit einer Druckgasflasche (8) für Sauerstoff und einem zwischen der Druckgasflasche (8) und dem Abgabeelement (11) angeordneten Dosierelement (9) versehen ist sowie bei der das Dosierelement (9) an eine Steuerung (12) zur Durchführung einer Oxigenierung angeschlossen ist, **dadurch gekennzeichnet, daß** die Steuerung (6) eine Zeitsynchronisation zur zeitlich aufeinander folgenden Durchführung von Inspirationsphasen und Exspirationsphasen, einer Fibrillationsanalyse sowie einer anschließenden Defibrillation bei Erkennung eines Fibrillations-Zustandes aufweist.

2. Vorrichtung nach Anspruch 1, **dadurch gekennzeichnet, daß** das Dosierelement (9) als ein Durchflußbegrenzer ausgebildet ist.

3. Vorrichtung nach Anspruch 1 oder 2, **dadurch gekennzeichnet, daß** das Dosierelement (9) auf eine Durchflußmenge von etwa 1000ml bis 1500ml je Minute an reinem Sauerstoff eingestellt ist.

4. Vorrichtung nach einem der Ansprüche 1 bis 3, **dadurch gekennzeichnet, daß** das Abgabeelement (11) als eine Nasalmaske ausgebildet ist.

5. Vorrichtung nach einem der Ansprüche 1 bis 3, **dadurch gekennzeichnet, daß** das Abgabeelement (11) als eine Sauerstoffbrille ausgebildet ist.

6. Vorrichtung nach einem der Ansprüche 1 bis 5, **dadurch gekennzeichnet, daß** der Defibrillator (1) einen Detektor (16) aufweist, der an die Elektroden (4, 5) angeschlossen ist.

7. Vorrichtung nach einem der Ansprüche 1 bis 6, **dadurch gekennzeichnet, daß** sowohl der Defibrillator (1) als auch eine Steuerung (12) für die Druckgasflasche (8) an einen übergeordneten Koordinator (17) angeschlossen sind.

8. Vorrichtung nach einem der Ansprüche 1 bis 7, **dadurch gekennzeichnet, daß** zur Druckerzeugung ein Beatmungsbeutel (21) verwendet ist.

9. Vorrichtung nach einem der Ansprüche 1 bis 8, **dadurch gekennzeichnet, daß** die Steuerung (12) eine zeitliche Taktung zur Vorgabe aufeinander folgender Inspirationsphasen sowie Exspirationsphasen aufweist.

10. Vorrichtung nach einem der Ansprüche 1 bis 9, **dadurch gekennzeichnet, daß** die Steuerung (6) eine zeitliche Synchronisierung zur Durchführung einer Fibrillationsanalyse im Anschluß an die Durchführung von Inspirationsphasen und Exspirationsphasen aufweist.

## Claims

1. Defibrillation device comprising a voltage generator (13), at least two electrodes (4, 5) connected to the voltage generator (13), a control for coordinating functions and a respirator unit with an output element, which respirator unit is provided with a pressure cylinder (8) for oxygen and a metering element (9) disposed between the pressure cylinder (8) and the output element (11), the metering element (9) being connected to a control (12) for providing a supply of oxygen, **characterised in that** the control (6) has a time synchroniser system for running consecutively timed inspiration phases and expiration phases, a fibrillation analysis and then defibrillation if a fibrillation state is detected.

2. Device as claimed in claim 1, **characterised in that** the metering element (9) is a flow restrictor.

3. Device as claimed in claim 1 or 2, **characterised in that** the metering element (9) is set to deliver a quantity of approximately 1000 ml to 1500 ml per minute of pure oxygen.

4. Device as claimed in one of claims 1 to 3, **characterised in that** the output element (11) is a nasal mask.

5. Device as claimed in one of claims 1 to 3, **characterised in that** the output element (11) is a set of oxygen goggles.

6. Device as claimed in one of claims 1 to 5, **characterised in that** the defibrillator (1) has a detector (16) which is connected to the electrodes (4, 5).

7. Device as claimed in one of claims 1 to 6, **characterised in that** both the defibrillator (1) and a control (12) for the pressure cylinder (8) are connected to a primary co-ordinator (17).

8. Device as claimed in one of claims 1 to 7, **characterised in that** a respirator bag (21) is provided as a means of generating pressure.

9. Device as claimed in one of claims 1 to 8, **characterised in that** the control (12) has a timing system for pre-setting consecutive inspiration and expiration phases.

10. Device as claimed in one of claims 1 to 9, **characterised in that** the control (6) has a time synchroniser for running a fibrillation analysis in conjunction with running the inspiration phases and expiration phases.

## Revendications

1. Dispositif de défibrillation, qui comprend un générateur de tension (13), au moins deux électrodes (4, 5) reliées au générateur de tension (13), une commande pour la coordination fonctionnelle, de même qu'un dispositif respiratoire avec un organe de délivrance, et dans lequel le dispositif respiratoire est pourvu d'un récipient (8) de gaz sous pression, pour de l'oxygène, et d'un organe de dosage (9) agencé entre le récipient pour gaz sous pression (8) et l'organe de délivrance (11), et dans lequel l'organe de dosage (9) est raccordé à une commande (12) pour réaliser une alimentation en oxygène, **caractérisé en ce que** la commande (6) présente une synchronisation temporelle pour produire en succession temporelle des phases d'inspiration et d'expiration, une analyse de la fibrillation de même qu'une défibrillation en conséquence de la détection d'un état fibrillatoire.

2. Dispositif selon la revendication 1, **caractérisé en ce que** l'organe de dosage (9) est réalisé sous la forme d'un limiteur de débit.

3. Dispositif selon la revendication 1 ou 2, **caractérisé en ce que** l'organe de dosage (9) est réglé pour un débit d'environ 1000 ml à 1500 ml d'oxygène pur par minute.

4. Dispositif selon l'une des revendications 1 à 3, **caractérisé en ce que** l'organe de délivrance (11) est réalisé sous la forme d'un masque nasal.

5. Dispositif selon l'une des revendications 1 à 3, **caractérisé en ce que** l'organe de délivrance (11) est réalisé sous la forme de lunettes à oxygène.

6. Dispositif selon l'une des revendications 1 à 5, **caractérisé en ce que** le défibrillateur (1) présente un détecteur (16) qui est relié aux électrodes (4, 5).

7. Dispositif selon l'une des revendications 1 à 6, **caractérisé en ce que**, aussi bien le défibrillateur (1) qu'une commande (12) pour le récipient (8) pour gaz sous pression sont raccordés à un coordinateur (17) de niveau supérieur.

8. Dispositif selon l'une des revendications 1 à 7, **caractérisé en ce qu'**un ballon respiratoire (21) est utilisé pour la production de pression.

9. Dispositif selon l'une des revendications 1 à 8, **caractérisé en ce que** la commande (12) présente un cadençage dans le temps pour donner des phases d'inspiration de même que des phases d'expiration qui se succèdent.

10. Dispositif selon l'une des revendications 1 à 9, **caractérisé en ce que** la commande (6) présente une synchronisation temporelle pour effectuer une analyse de fibrillation conjointement à la réalisation de phases d'inspiration et de phases d'expiration.
